# EUROPEAN PATENT SPECIFICATION

(11) **EP 3 709 980 B1**
(45) Date of publication and mention of the grant of the patent: **10.01.2024**
(21) Application number: 18879750.0
(22) Date of filing: 12.11.2018
(51) Int. Cl.: A61K 9/70

(54) **MICROCELL SYSTEMS FOR DELIVERING HYDROPHILIC ACTIVE MOLECULES**
MIKROZELLENSYSTEME ZUR ABGABE VON HYDROPHILEN AKTIVEN MOLEKÜLEN
SYSTÈMES DE MICROCELLULES POUR L'ADMINISTRATION DE MOLÉCULES ACTIVES HYDROPHILES

(30) Priority: 14.11.2017 US 201762585674 P
(43) Date of publication of application: 23.09.2020
(73) Proprietor: E Ink Corporation, Billerica, MA 01821 (US)
(72) Inventor: LIU, Lei, Fremont, California 94538 (US)
(74) Representative: Kilburn & Strode LLP
(86) International application number: PCT/US2018/060266
(87) International publication number: WO 2019/099323

(56) References cited:
- WO-A1-2018/175825
- WO-A2-2005/094526
- US-A1- 2009 234 214
- US-A1- 2012 176 663
- US-A1- 2015 005 720
- US-A1- 2016 279 072
- US-A1- 2018 271 800

## Description

### BACKGROUND

Hydrophilic active molecules, such as vitamins, antibiotics (e.g., penicillin), and salts of certain compounds, are often stabilized in a matrix or gel for delivery with a transdermal system. Matrices and gels require a large quantity of non-active materials (e.g., cellulose, cyclodextrin, polyethylene oxide), and the amount of hydrophilic active that can be captured into, and released from, the matrix may be limited. Additionally, the active molecules may crystalize within the matrix during storage, limiting the shelf life of the delivery system. Because of these limitations, the amount of hydrophilic active that can be delivered with a "standard" amount of matrix or gel may not be sufficient for all patients. Consequently, if a "high" dose is required, a physician will direct a patient to place multiple matrix-containing transdermal patches, or direct the patient to apply the gel several times during the day. A system that allows more variability in the concentrations of these actives, as well as more precise control of the release profile, is desirable.

US 2015/005720 A1 describes a transdermal delivery system in the form of a film comprising one or more microcups filled with a liquid composition (which may be aqueous) containing an active ingredient. The microcups are sealed with a sealing layer (diffusion membrane) which is preferably a microporous film, and the delivery system also comprises a skin contact layer which adheres to the skin of a patient. Different microcups may be filled with different active ingredients.

WO 2018/175825 A1 describes an active molecule delivery system whereby active molecules can be released on demand and/or a variety of different active molecules can be delivered from the same system and/or different concentrations of active molecules can be delivered from the same system. The active delivery system includes a plurality of microcells, wherein the microcells are filled with a medium including active molecules. The microcells include an opening, and the opening is spanned by a porous diffusion layer. The microcell arrays may be loaded with different active ingredients, thereby providing a mechanism to deliver different, or complimentary, active ingredients on demand.

### SUMMARY

The invention addresses these needs by providing a transdermal delivery system whereby hydrophilic active molecules can be prepared in simple aqueous solutions and then delivered transdermally. Furthermore, the systems of the invention allow for the delivery of different types, and/or concentrations, and/or volumes of hydrophilic active molecules from the same delivery system.

Thus, the invention provides an active molecule delivery system in accordance with the appended claims.

The microcells each include includes walls and an opening. The microcells may be square, round, or polygonal, such as a honeycomb structure. For each microcell, the opening is spanned the hydrophobic sealing layer. The hydrophobic sealing layer may be constructed from a variety of materials, such as polyisobutylene, a polyethylene, a polyurethane, a polycaprolactone, or a polysiloxane. The hydrophobic sealing layer is spanned by a porous diffusion layer. The porous diffusion layer may be constructed from a variety of materials, such as acrylate, methacrylate, polycarbonate, polyvinyl alcohol, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polypropylene, polybutylene, polyisobutylene, or polystyrene. Typically, each microcell has a volume greater than 100 nL. In some embodiments, the porous diffusion layer has an average pore size of between 1 nm and 100 nm. The hydrophilic actives can be any active that is soluble or partially soluble in water, including pharmaceutical compounds, aroma compounds (e.g., perfumes), nucleic acids (e.g., DNA, RNA), or amino acids (e.g., proteins, e.g., vaccines, antibodies, or enzymes).

The system includes first and second microcells, wherein the first microcell includes a first aqueous formulation of a first hydrophilic active molecule and the second microcell includes a second aqueous formulation of a second hydrophilic active molecule. The first and second active molecules may be different. In another embodiment, the first microcell includes a first concentration of a formulation including a hydrophilic active molecule and the second microcell includes a second concentration of the same formulation, wherein the first and second concentrations are different. The first microcell has a first porous diffusion layer with a first porosity and the second microcell has a second porous diffusion layer with a second porosity, wherein the first and second porosities are different. In addition to varying the type and concentration of active molecules, it is also possible to prepare a system including an active and another useful compound such as a vitamin, adjuvant, etc. Other combinations of active molecules, agents, and concentrations will be evident to one of skill in the art.

In some embodiments, the aqueous formulations will include additional components such as thickening agents, colorants, adjuvants, vitamins, salts, or buffering agents. The mixtures may also include charge control agents, surfactants, and preservatives.

### BRIEF DESCRIPTION OF THE DRAWINGS

FIG. 1 (which is not an embodiment of the invention) illustrates a hydrophilic active molecule delivery system including a plurality of microcells formed from a thermoplastic material and containing an aqueous formulation including a hydrophilic active molecule. The microcells are sealed with a hydrophobic sealing layer, and the system additionally includes an adhesive layer;
FIG. 2 (which is not an embodiment of the invention) illustrates an active molecule delivery system including a plurality of microcells, a hydrophobic sealing layer, and a porous diffusion layer. In the embodiment of FIG. 2 different microcells include different active molecules;
FIG. 3 illustrates an embodiment of an active molecule delivery system including a plurality of microcells, a hydrophobic sealing layer, and a porous diffusion layer. In the embodiment of FIG. 3, different microcells include different porosities in the porous diffusion layer and thus have different delivery profiles;
FIG. 4 shows a method for making microcells for the invention using a roll-to-roll process;
FIGS. 5A and 5B detail the production of microcells for an active molecule delivery system using photolithographic exposure through a photomask of a conductor film coated with a thermoset precursor;
FIGS. 5C and 5D detail an alternate embodiment in which microcells for an active molecule delivery system are fabricated using photolithography. In FIGS. 5C and 5D a combination of top and bottom exposure is used, allowing the walls in one lateral direction to be cured by top photomask exposure, and the walls in another lateral direction to be cured bottom exposure through the opaque base conductor film;
FIGS. 6A-6D illustrate the steps of filling and sealing an array of microcells to be used in an active molecule delivery system;
FIG. 7 is a microscopic image of a layer of microcells filled with an aqueous formulation that is dyed with blue food coloring. The cells are sealed with a hydrophobic sealing layer comprising polyisobutylene;
FIG. 8 shows a microscope image of polyisobutylene sealing layer and the porous diffusion layer formed from an acrylic/methacrylic acid copolymer (EUDRAGIT^{®}, Evonik, Essen, DE).

### DESCRIPTION

The invention provides a hydrophilic active molecule delivery system whereby active molecules can be released on demand and/or a variety of different active molecules can be delivered from the same system and/or different concentrations of active molecules can be delivered from the same system. The invention is well-suited for delivering hydrophilic pharmaceuticals to patients transdermally, however the invention may be used to deliver hydrophilic active ingredients, generally. For example, the invention can be used to deliver larger molecules that need to be kept in an aqueous buffered environment, such as enzymes or antibodies. The active delivery system includes a plurality of microcells, wherein the microcells are filled with a medium including the hydrophilic active molecules. The microcells include an opening, and the opening is spanned by a hydrophobic sealing layer. The sealing layer is overcoated with a porous diffusion layer.

In addition to more conventional applications, such as transdermal delivery of pharmaceutical compounds, the active molecule delivery system may be the basis for delivering agricultural nutrients. For example, the microcell arrays can be fabricated into large sheets that can be used in conjunction with hydroponic growing systems, or the microcell arrays can be integrated into hydrogel film farming. See, for example, Mebiol, Inc. (Kanagawa, Japan). The active molecule delivery systems can also be incorporated into the structural walls of smart packing. Such delivery systems makes it possible to have long term release of antioxidants into a package containing fresh vegetables. This "smart" packaging will dramatically improve the shelf life of certain foods, and it will only require the amount of antioxidant necessary to maintain freshness until the package is opened. Thus, the same packaging can be used for food that is distributed locally, across the country, or around the globe.

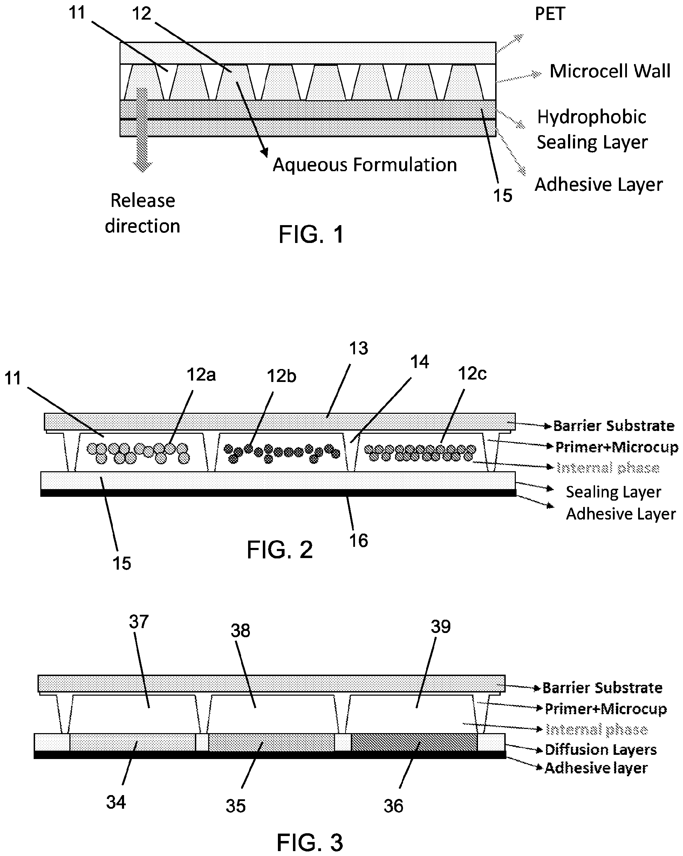

An overview of a hydrophilic active molecule delivery system is shown in FIG. 1. The system includes a plurality of microcells 11, each microcell including an aqueous medium (a.k.a. internal phase), that includes hydrophilic active molecules 12. Each microcell includes a wall and an opening which is sealed with a hydrophobic sealing layer 15, which may be constructed from, e.g., polyisobutylene, a polyethylene, a polyurethane, a polycaprolactone, or a polysiloxane. The delivery system additionally includes a biocompatible adhesive, which may include, for example, a polyisobutylene, an acrylic, a poly(ethylene)glycol, or a silicone.

As shown in FIG. 2, a first microcell may include a first hydrophilic active 12a, while a second microcell includes a second hydrophilic active 12b, while a third microcell includes a third hydrophilic active 12c. Each microcell 11 is part of an array that is formed from a polymer matrix, which is described in more detail below. The active molecule delivery system will typically include a backing barrier 13 to provide structural support and protection against moisture ingress and physical interactions. The microcells are defined by walls 14 that are at least 1 µm high, although they can be much higher depending upon the desired depth of the microcell. The microcells may be arranged as squares, a honeycomb, circles, etc. Often the system will additionally include an adhesive layer 16 that is also porous to the active molecule. The adhesive layer 16 assists in keeping the active molecule delivery system adjacent to the surface.

The delivery system includes porous diffusion layers, which may be constructed from a variety of natural or non-natural polymers, such as acrylates, methacrylates, polycarbonates, polyvinyl alcohols, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polypropylene, polybutylene, polyisobutylene, or polystyrene. Using picoliter injection with inkjet or other fluidic systems, individual microcells can be filled to enable a variety of different actives to be included in an active molecule delivery system.

FIG. 3 shows an embodiment of a hydrophilic active molecule delivery system in which the porosity of the diffusion layer is varied for different microcells. This can be accomplished by using different polymer materials and microinjection, e.g., using inkjet during the sealing process (described below). Such systems allow a single delivery system to administer varying concentrations of the same or different active molecules over a period of time. For example, a system of the invention may include three microcells 37, 38, 39 with folic acid at three different concentrations. However, the dosage time will be controlled by the porosity of the diffusion layer. For example, shortly after application the most concentrated dose may be delivered via the first microcell 37 via the most porous diffusion layer 34, followed by a maintenance dose delivered from the second microcell 38, and then during the nighttime, the least concentrated dosage will be delivered via the third microcell 39 via the least porous diffusion layer 36.

Of course, a variety of combinations are possible, and varying microcells might include hydrophilic pharmaceuticals, hydrophilic nutraceuticals, hydrophilic adjuvants, hydrophilic vitamins, or vaccines. Furthermore, the arrangement of the microcells may not be distributed. Rather the microcells may be filled in clusters, which makes filling and sealing more straightforward. In other embodiments, smaller microcell arrays may be filled with the same medium, i.e., having the same active molecule at the same concentration, and then the smaller arrays assembled into a larger array to make a delivery system of the invention. In other embodiments, differing porosity can be used with differing contents for a microcell. For example, one microcell may include a solution of enzymes and require a porous diffusion layer that has pores large enough for the enzymes to pass, while an adjacent microcell may include a substrate to activate the enzyme, but require a porous diffusion layer with much smaller pores to regulate delivery of the substrate.

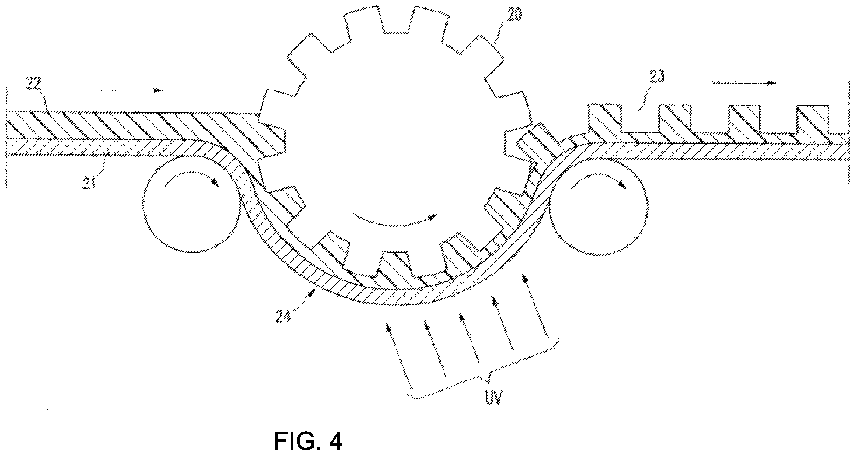

**Techniques for constructing microcells.** Microcells may be formed either in a batchwise process or in a continuous roll-to-roll process as disclosed in U.S. Pat. No. 6,933,098. The latter offers a continuous, low cost, high throughput manufacturing technology for production of compartments for use in a variety of applications including active molecule delivery and electrophoretic displays. Microcell arrays suitable for use with the invention can be created with microembossing, as illustrated in FIG. 4. A male mold 20 may be placed either above the web 24, as shown in FIG.4, or below the web 24 (not shown) however alternative arrangements are possible. See U.S. Patent No. 7,715,088. A conductive substrate may be constructed by forming a conductor film 21 on polymer substrate that becomes the backing for a device. A composition comprising a thermoplastic, thermoset, or a precursor thereof 22 is then coated on the conductor film. The thermoplastic or thermoset precursor layer is embossed at a temperature higher than the glass transition temperature of the thermoplastics or thermoset precursor layer by the male mold in the form of a roller, plate or belt.

The thermoplastic or thermoset precursor for the preparation of the microcells may be multifunctional acrylate or methacrylate, vinyl ether, epoxide and oligomers or polymers thereof, and the like. A combination of multifunctional epoxide and multifunctional acrylate is also very useful to achieve desirable physico-mechanical properties. A crosslinkable oligomer imparting flexibility, such as urethane acrylate or polyester acrylate, may be added to improve the flexure resistance of the embossed microcells. The composition may contain polymer, oligomer, monomer and additives or only oligomer, monomer and additives. The glass transition temperatures (or T_{g}) for this class of materials usually range from about -70° C. to about 150° C., preferably from about -20° C. to about 50° C. The microembossing process is typically carried out at a temperature higher than the T_{g}. A heated male mold or a heated housing substrate against which the mold presses may be used to control the microembossing temperature and pressure.

As shown in FIG. 4, the mold is released during or after the precursor layer is hardened to reveal an array of microcells 23. The hardening of the precursor layer may be accomplished by cooling, solvent evaporation, cross-linking by radiation, heat or moisture. If the curing of the thermoset precursor is accomplished by UV radiation, UV may radiate onto the transparent conductor film from the bottom or the top of the web as shown in the two figures. Alternatively, UV lamps may be placed inside the mold. In this case, the mold must be transparent to allow the UV light to radiate through the pre-patterned male mold on to the thermoset precursor layer. A male mold may be prepared by any appropriate method, such as a diamond turn process or a photoresist process followed by either etching or electroplating. A master template for the male mold may be manufactured by any appropriate method, such as electroplating. With electroplating, a glass base is sputtered with a thin layer (typically 3000 Å) of a seed metal such as chrome inconel. The mold is then coated with a layer of photoresist and exposed to UV. A mask is placed between the UV and the layer of photoresist. The exposed areas of the photoresist become hardened. The unexposed areas are then removed by washing them with an appropriate solvent. The remaining hardened photoresist is dried and sputtered again with a thin layer of seed metal. The master is then ready for electroforming. A typical material used for electroforming is nickel cobalt. Alternatively, the master can be made of nickel by electroforming or electroless nickel deposition. The floor of the mold is typically between about 50 to 400 microns. The master can also be made using other microengineering techniques including e-beam writing, dry etching, chemical etching, laser writing or laser interference as described in "Replication techniques for micro-optics", SPIE Proc. Vol. 3099, pp. 76-82 (1997). Alternatively, the mold can be made by photomachining using plastics, ceramics or metals.

Prior to applying a UV curable resin composition, the mold may be treated with a mold release to aid in the demolding process. The UV curable resin may be degassed prior to dispensing and may optionally contain a solvent. The solvent, if present, readily evaporates. The UV curable resin is dispensed by any appropriate means such as, coating, dipping, pouring or the like, over the male mold. The dispenser may be moving or stationary. A conductor film is overlaid the UV curable resin. Pressure may be applied, if necessary, to ensure proper bonding between the resin and the plastic and to control the thickness of the floor of the microcells. The pressure may be applied using a laminating roller, vacuum molding, press device or any other like means. If the male mold is metallic and opaque, the plastic substrate is typically transparent to the actinic radiation used to cure the resin. Conversely, the male mold can be transparent and the plastic substrate can be opaque to the actinic radiation. To obtain good transfer of the molded features onto the transfer sheet, the conductor film needs to have good adhesion to the UV curable resin which should have a good release property against the mold surface.

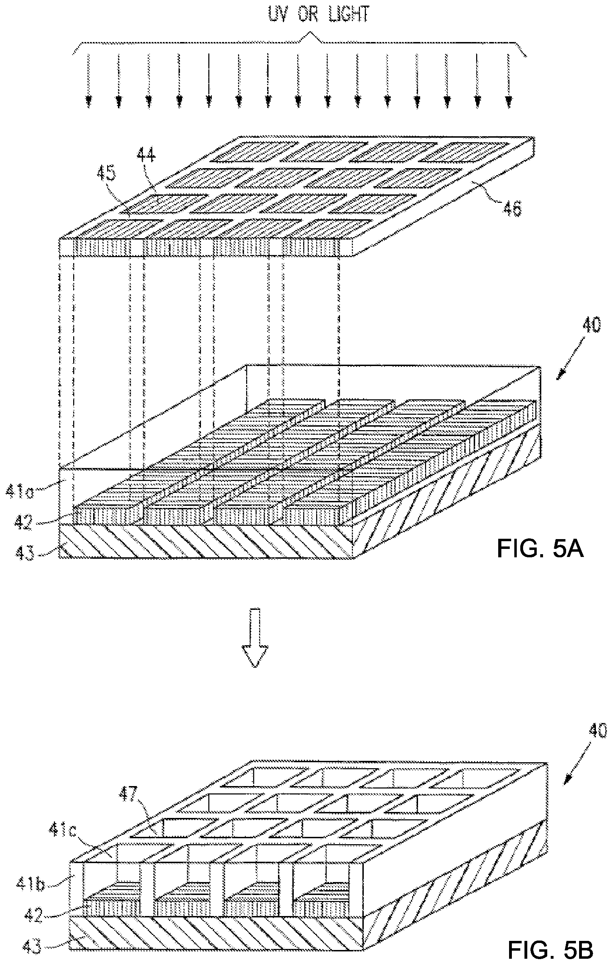

**Photolithography.** Microcells can also be produced using photolithography. Photolithographic processes for fabricating a microcell array are illustrated in FIGS. 5A and 5B. As shown in FIGS. 5A and 5B, the microcell array 40 may be prepared by exposure of a radiation curable material 41a coated by known methods onto a conductor electrode film 42 to UV light (or alternatively other forms of radiation, electron beams and the like) through a mask 46 to form walls 41b corresponding to the image projected through the mask 46. The base conductor film 42 is preferably mounted on a supportive substrate base web 43, which may comprise a plastic material.

In the photomask 46 in FIG. 5A, the dark squares 44 represent the opaque area and the space between the dark squares represents the transparent area 45 of the mask 46. The UV radiates through the transparent area 45 onto the radiation curable material 41a. The exposure is preferably performed directly onto the radiation curable material 41a, i.e., the UV does not pass through the substrate 43 or base conductor 42 (top exposure). For this reason, neither the substrate 43, nor the conductor 42, needs to be transparent to the UV or other radiation wavelengths employed.

As shown in FIG. 5B, the exposed areas 41b become hardened and the unexposed areas (protected by the opaque area 44 of the mask 46) are then removed by an appropriate solvent or developer to form the microcells 47. The solvent or developer is selected from those commonly used for dissolving or reducing the viscosity of radiation curable materials such as methylethylketone (MEK), toluene, acetone, isopropanol or the like. The preparation of the microcells may be similarly accomplished by placing a photomask underneath the conductor film/substrate support web and in this case the UV light radiates through the photomask from the bottom and the substrate needs to be transparent to radiation.

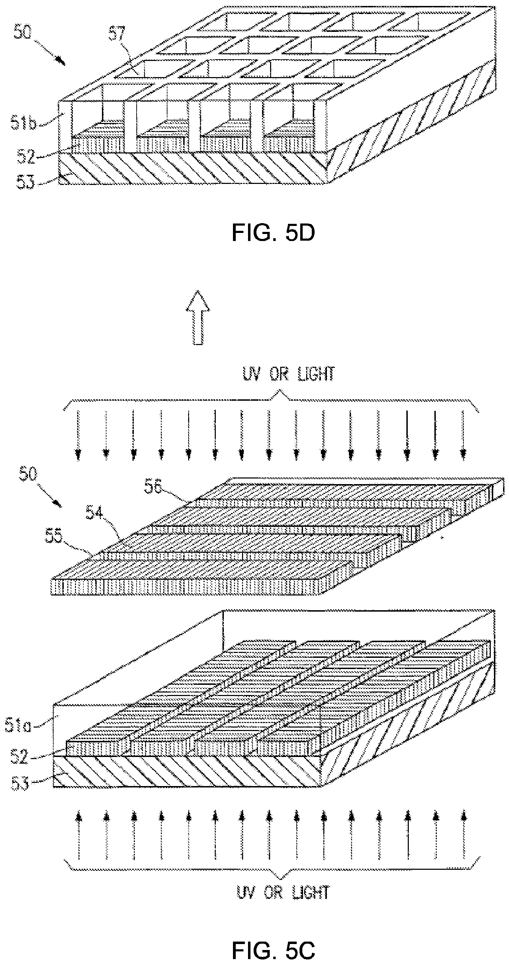

**Imagewise Exposure.** Still another alternative method for the preparation of the microcell array of the invention by imagewise exposure is illustrated in FIGS. 5C and 5D. When opaque conductor lines are used, the conductor lines can be used as the photomask for the exposure from the bottom. Durable microcell walls are formed by additional exposure from the top through a second photomask having opaque lines perpendicular to the conductor lines. FIG. 5C illustrates the use of both the top and bottom exposure principles to produce the microcell array 50 of the invention. The base conductor film 52 is opaque and line-patterned. The radiation curable material 51a, which is coated on the base conductor 52 and substrate 53, is exposed from the bottom through the conductor line pattern 52 which serves as the first photomask. A second exposure is performed from the "top" side through the second photomask 56 having a line pattern perpendicular to the conductor lines 52. The spaces 55 between the lines 54 are substantially transparent to the UV light. In this process, the wall material 51b is cured from the bottom up in one lateral orientation, and cured from the top down in the perpendicular direction, joining to form an integral microcell 57. As shown in FIG. 5D, the unexposed area is then removed by a solvent or developer as described above to reveal the microcells 57. The technique described in FIGS. 5C and 5D thus allow the different walls to be constructed with different porosity, as needed for the embodiment illustrated in FIG. 3.

The microcells may be constructed from thermoplastic elastomers, which have good compatibility with the microcells and do not interact with the electrophoretic media. Examples of useful thermoplastic elastomers include ABA, and (AB)n type of di-block, tri-block, and multi-block copolymers wherein A is styrene, α-methylstyrene, ethylene, propylene or norbonene; B is butadiene, isoprene, ethylene, propylene, butylene, dimethylsiloxane or propylene sulfide; and A and B cannot be the same in the formula. The number, n, is ≧1, preferably 1-10. Particularly useful are di-block or tri-block copolymers of styrene or ox-methylstyrene such as SB (poly(styrene-b-butadiene)), SBS (poly(styrene-b-butadiene-b-styrene)), SIS (poly(styrene-b-isoprene-b-styrene)), SEBS (poly(styrene-b-ethylene/butylenes-b-stylene)) poly(styrene-b-dimethylsiloxane-b-styrene), poly((α-methylstyrene-b-isoprene), poly(α-methylstyrene-b-isoprene-b-α-methylstyrene), poly(α-methylstyrene-b-propylene sulfide-b-α-methylstyrene), poly(α-methylstyrene-b-dimethylsiloxane-b-α-methylstyrene). Commercially available styrene block copolymers such as Kraton D and G series (from Kraton Polymer, Houston, Tex.) are particularly useful. Crystalline rubbers such as poly(ethylene-co-propylene-co-5-methylene-2-norbomene) or EPDM (ethylene-propylene-diene terpolymer) rubbers such as Vistalon 6505 (from Exxon Mobil, Houston, Tex.) and their grafted copolymers have also been found very useful.

The thermoplastic elastomers may be dissolved in a solvent or solvent mixture which is immiscible with the display fluid in the microcells and exhibits a specific gravity less than that of the display fluid. Low surface tension solvents are preferred for the overcoating composition because of their better wetting properties over the microcell walls and the electrophoretic fluid. Solvents or solvent mixtures having a surface tension lower than 35 dyne/cm are preferred. A surface tension of lower than 30 dyne/cm is more preferred. Suitable solvents include alkanes (preferably C₆₋₁₂ alkanes such as heptane, octane or Isopar solvents from Exxon Chemical Company, nonane, decane and their isomers), cycloalkanes (preferably C₆₋₁₂ cycloalkanes such as cyclohexane and decalin and the like), alkylbezenes (preferably mono- or di-C₁₋₆ alkyl benzenes such as toluene, xylene and the like), alkyl esters (preferably C₂₋₅ alkyl esters such as ethyl acetate, isobutyl acetate and the like) and C₃₋₅ alkyl alcohols (such as isopropanol and the like and their isomers). Mixtures of alkylbenzene and alkane are particularly useful.

In addition to polymer additives, the polymer mixtures may also include wetting agents (surfactants). Wetting agents (such as the FC surfactants from 3M Company, Zonyl fluorosurfactants from DuPont, fluoroacrylates, fluoromethacrylates, fluoro-substituted long chain alcohols, perfluoro-substituted long chain carboxylic acids and their derivatives, and Silwet silicone surfactants from OSi, Greenwich, Conn.) may also be included in the composition to improve the adhesion of the sealant to the microcells and provide a more flexible coating process. Other ingredients including crosslinking agents (e.g., bisazides such as 4,4'-diazidodiphenylmethane and 2,6-di-(4'-azidobenzal)-4-methylcyclohexanone), vulcanizers (e.g., 2-benzothiazolyl disulfide and tetramethylthiuram disulfide), multifunctional monomers or oligomers (e.g., hexanediol, diacrylates, trimethylolpropane, triacrylate, divinylbenzene, diallylphthalene), thermal initiators (e.g., dilauroryl peroxide, benzoyl peroxide) and photoinitiators (e.g., isopropyl thioxanthone (ITX), Irgacure 651 and Irgacure 369 from Ciba-Geigy) are also highly useful to enhance the physico-mechanical properties of the sealing layer by crosslinking or polymerization reactions during or after the overcoating process.

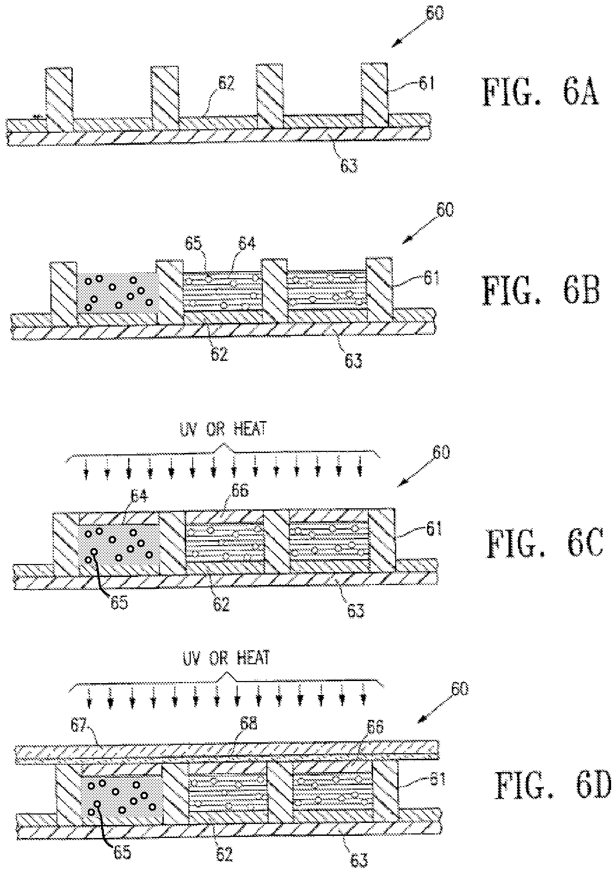

After the microcells are produced, they are filled with appropriate mixtures of active molecules. The microcell array 60 may be prepared by any of the methods described above. As shown in cross-section in FIGS. 6A-6D, the microcell walls 61 extend upward from the substrate 63 to form the open cells. The microcells may include a primer layer 62 to passivate the mixture and keep the microcell material from interacting with the mixture containing the actives 65. Prior to filling, the microcell array 60 may be cleaned and sterilized to assure that the active molecules are not compromised prior to use.

The microcells are next filled with a mixture 64 including active molecules 65. As shown in FIG. 6B, different microcells include different actives. The microcells 60 are preferably partially filled to prevent overflow and the unintentional mixing of active ingredients. In systems for delivering hydrophilic active molecules, the mixture may be based upon water or another aqueous medium such as phosphate buffer.

The microcells may be filled using a variety of techniques. In some embodiments, where a large number of neighboring microcells are to be filled with an identical mixture, blade coating may be used to fill the microcells to the depth of the microcell walls 61. In other embodiments, where a variety of different mixtures are to be filled in a variety of nearby microcell, inkjet-type microinjection can be used to fill the microcells. In yet other embodiments, microneedle arrays may be used to fill an array of microcells with the correct mixtures. The filling may be done in a one-step, or a multistep process. For example, all of the cells may be partially filled with an amount of solvent. The partially filled microcells are then filled with a second mixture including the one or more active molecules to be delivered.

As shown in FIG. 6C, after filling, the microcells are sealed by applying a polymer 66 that becomes the porous diffusion layer. In some embodiments, the sealing process may involve exposure to heat, dry hot air, or UV radiation. In most embodiments the polymer 66 will be compatible with the mixture 64, but not dissolved by the solvent of the mixture 64. The polymer 66 will also be biocompatible and selected to adhere to the sides or tops of the microcell walls 61. A suitable biocompatible adhesive for the porous diffusion layer is a phenethylamine mixture, such as described in U.S. Patent No. 10,087,344. Accordingly, the final microcell structure is mostly impervious to leaks and able to withstand flexing without delamination of the porous diffusion layer.

In alternate embodiments, a variety of individual microcells may be filled with the desired mixture by using iterative photolithography. The process typically includes coating an array of empty microcells with a layer of positively working photoresist, selectively opening a certain number of the microcells by imagewise exposing the positive photoresist, followed by developing the photoresist, filling the opened microcells with the desired mixture, and sealing the filled microcells by a sealing process. These steps may be repeated to create sealed microcells filled with other mixtures. This procedure allows for the formation of large sheets of microcells having the desired ratio of mixtures or concentrations.

After the microcells 60 are filled, the sealed array may be laminated with a finishing layer 68 that is also porous to the active molecules, preferably by pre-coating the finishing layer 68 with an adhesive layer which may be a pressure sensitive adhesive, a hot melt adhesive, or a heat, moisture, or radiation curable adhesive. The laminate adhesive may be post-cured by radiation such as UV through the top conductor film if the latter is transparent to the radiation. In some embodiments, a biocompatible adhesive 67 is then laminated to the assembly. The biocompatible adhesive 67 will allow active molecules to pass through while keeping the device mobile on a user. Suitable biocompatible adhesives are available from 3M (Minneapolis, MN).

Once the delivery system has been constructed, it may be covered with an encapsulating covering to provide protection against physical shock. The encapsulating covering may also include adhesives to make sure that the active molecule delivery system stays affixed, e.g., to a patient's back. The encapsulating covering may also include aesthetic coloring or fun designs for children.

### EXAMPLE - Microcell assembly filled with aqueous solution (not according to the invention)

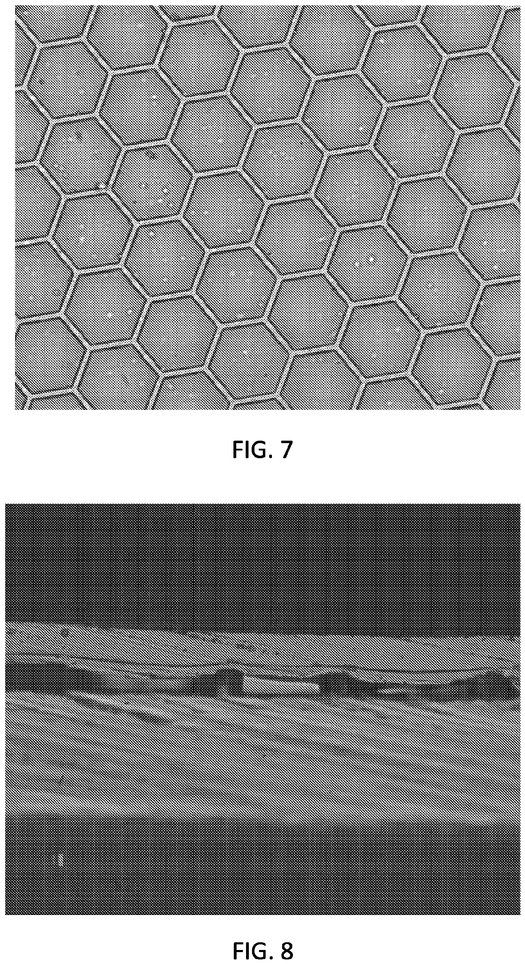

A hydrophilic molecule delivery system including microcells, a hydrophobic sealing layer, and a porous diffusion layer was constructed. A microcell layer was prepared by microembossing polyethylene terephthalate (PET) as described above. Next, a 5% aqueous solution of ethylene vinyl alcohol copolymer (RS1717 from Kuraray) in D.I. water was prepared. To improve visualization, blue food coloring was added to the 5% polymer solution. The microcells were filled with the colored 5% solution using a pipette, and the remnant solution was removed with a rubber spatula. The filled microcells were overcoated with a solution of polyisobutylene (PIB) in xylene. The PIB in the sealing layer had an average molecular weight of 850KD. The xylene was allowed to evaporate, thereby creating a hydrophobic sealing layer. A microscope view of the filled and sealed microcell layer is shown in FIG. 7.

After the sealing layer was cured, a porous diffusion layer was added on top of the sealing layer. The porous diffusion layer was made from Eudragit E100 (in MEK), a commercial copolymer comprising dimethylaminoethyl methacrylate, butyl methacrylate, and methyl methacrylate, available from Evonic GmbH (Essen, Germany). As shown in FIG.8, the Eudragit, together with PIB, produces a uniform barrier with a well-defined aqueous volume inside the microcell. While not shown here, an adhesive layer may be applied directly to the porous diffusion layer to facilitate long-term placement of the active molecule delivery system.

## Claims

1. An active molecule delivery system comprising:
a plurality of thermoplastic microcells (37, 38, 39) filled with an aqueous formulation comprising hydrophilic active molecules, wherein each microcell includes walls and an opening, the microcells including a first microcell (37) filled with a first aqueous formulation and a second microcell (38) filled with a second aqueous formulation different from the first aqueous formulation;
a hydrophobic sealing layer spanning the opening; and
a biocompatible adhesive,
the active molecule delivery system being **characterized by** first (34) and second (35) porous diffusion layers disposed between the hydrophobic sealing layer and the biocompatible adhesive, the first porous diffusion layer (34) being disposed adjacent the first microcell (37) and having a first porosity and the second porous diffusion layer (35) being disposed adjacent the second microcell (38) and having a second porosity different from the first porosity.

2. The active molecule delivery system of claim 1, wherein the hydrophobic sealing layer comprises a polyisobutylene, a polyethylene, a polyurethane, a polycaprolactone, or a polysiloxane.

3. The active molecule delivery system of claim 1, wherein the porous diffusion layer comprises an acrylate, a methacrylate, a polycarbonate, a polyvinyl alcohol, cellulose, poly(N-isopropylacrylamide) (PNIPAAm), poly(lactic-co-glycolic acid) (PLGA), polyvinylidene chloride, acrylonitrile, amorphous nylon, oriented polyester, terephthalate, polyvinyl chloride, polyethylene, polypropylene, polybutylene, polyisobutylene, or polystyrene.

4. The active molecule delivery system of claim 1, wherein each porous diffusion layer (34, 35, 36) has an average pore size of between 10 nm and 100 µm.

5. The active molecule delivery system of claim 1, wherein the hydrophilic active molecule is a pharmaceutical compound, an aroma compound, or comprises nucleic acids or amino acids.

6. The active molecule delivery system of claim 1, wherein each of the plurality of microcells has a volume greater than 100 nL.

7. The active molecule delivery system of claim 1, wherein the aqueous formulation comprises more than one type of hydrophilic active molecule.

8. The active molecule delivery system of claim 1, wherein the first and second aqueous formulations differ in the concentration of the active molecule.

9. The active molecule delivery system of claim 1, further comprising an encapsulating cover that encapsulates the active molecule delivery system.

10. The active molecule delivery system of claim 1, further comprising a backing layer in contact with the adhesive layer.

11. The active molecule delivery system of claim 1, wherein the aqueous formulation additionally includes a thickening agent.

12. The active molecule delivery system of claim 11, wherein the thickening agent is a polymer.

13. The active molecule delivery system of claim 12, wherein the polymer is an ethylene vinyl alcohol copolymer.

## Patentansprüche

1. Wirkstoffmolekülzuführungssystem, umfassend:
mehrere thermoplastische Mikrozellen (37, 38, 39), die mit einer wässrigen Formulierung gefüllt sind, die hydrophile Wirkstoffmoleküle umfasst, wobei jede Mikrozelle Wände und eine Öffnung aufweist, wobei die Mikrozellen eine erste Mikrozelle (37), die mit einer ersten wässrigen Formulierung gefüllt ist, und eine zweite Mikrozelle (38) die mit einer zweiten wässrigen Formulierung, die von der ersten wässrigen Formulierung verschieden ist, gefüllt ist, einschließen;
eine hydrophobe Versiegelungsschicht, die die Öffnung überspannt; und
einen biokompatiblen Klebstoff,
wobei das Wirkstoffmolekülzuführungssystem **gekennzeichnet ist durch** eine erste poröse Diffusionsschicht (34) und eine zweite poröse Diffusionsschicht (35), die zwischen der hydrophoben Versiegelungsschicht und dem biokompatiblen Klebstoff angeordnet sind, wobei die erste poröse Diffusionsschicht (34) an die erste Mikrozelle (37) angrenzend angeordnet ist und eine erste Porosität aufweist und die zweite poröse Diffusionsschicht (35), die an die zweite Mikrozelle (38) angrenzend angeordnet ist und eine zweite Porosität, die von der ersten Porosität verschieden ist, aufweist.

2. Wirkstoffmolekülzuführungssystem nach Anspruch 1, wobei die hydrophobe Versiegelungsschicht ein Polyisobutylen, ein Polyethylen, ein Polyurethan, ein Polycaprolacton oder ein Polysiloxan umfasst.

3. Wirkstoffmolekülzuführungssystem nach Anspruch 1, wobei die poröse Diffusionsschicht ein Acrylat, ein Methacrylat, ein Polycarbonat, einen Polyvinylalkohol, Cellulose, Poly(N-isopropylacrylamid) (PNIPAAm), Poly(milchsäure-co-glykolsäure) (PLGA), Polyvinylidenchlorid, Acrylnitril, amorphes Polyamid, orientierten Polyester, Terephthalat, Polyvinylchlorid, Polyethylen, Polypropylen, Polybutylen, Polyisobutylen oder Polystyrol umfasst.

4. Wirkstoffmolekülzuführungssystem nach Anspruch 1, wobei jede poröse Diffusionsschicht (34, 35, 36) eine durchschnittliche Porengröße zwischen 10 nm und 100 µm aufweist.

5. Wirkstoffmolekülzuführungssystem nach Anspruch 1, wobei das hydrophile Wirkstoffmolekül eine pharmazeutische Verbindung oder eine Aromaverbindung ist oder Nukleinsäuren oder Aminosäuren umfasst.

6. Wirkstoffmolekülzuführungssystem nach Anspruch 1, wobei jeder der mehreren Mikrozellen ein Volumen von mehr als 100 nl aufweist.

7. Wirkstoffmolekülzuführungssystem nach Anspruch 1, wobei die wässrige Formulierung mehr als einen Typ von hydrophilem Wirkstoffmolekül umfasst.

8. Wirkstoffmolekülzuführungssystem nach Anspruch 1, wobei sich die erste wässrige Formulierung und die zweite wässrige Formulierung hinsichtlich der Konzentration des Wirkstoffmoleküls unterscheiden.

9. Wirkstoffmolekülzuführungssystem nach Anspruch 1, ferner umfassend eine Einkapselungsabdeckung, die das Wirkstoffmolekülzuführungssystem einkapselt.

10. Wirkstoffmolekülzuführungssystem nach Anspruch 1, ferner umfassend eine Rückschicht in Kontakt mit der Klebstoffschicht.

11. Wirkstoffmolekülzuführungssystem nach Anspruch 1, wobei die wässrige Formulierung zusätzlich ein Verdickungsmittel enthält.

12. Wirkstoffmolekülzuführungssystem nach Anspruch 11, wobei es sich bei dem Verdickungsmittel um ein Polymer handelt.

13. Wirkstoffmolekülzuführungssystem nach Anspruch 12, wobei es sich bei dem Polymer um ein EthylenVinylalkohol-Copolymer handelt.

## Revendications

1. Système d'apport de molécules actives comprenant :
une pluralité de microcellules thermoplastiques (37, 38, 39) remplies avec une formulation aqueuse comprenant des molécules actives hydrophiles, chaque microcellule comprenant des parois et une ouverture, les microcellules comprenant une première microcellule (37) remplie avec une première formulation aqueuse et une deuxième microcellule (38) remplie avec une deuxième formulation aqueuse différente de la première formulation aqueuse ; une couche de scellement hydrophobe enjambant l'ouverture ; et
un adhésif biocompatible,
le système d'apport de molécules actives étant **caractérisé par** une première (34) et une deuxième (35) couche de diffusion poreuse disposées entre la couche de scellement hydrophobe et l'adhésif biocompatible, la première couche de diffusion poreuse (34) étant disposée adjacente à la première microcellule (37) et possédant une première porosité et la deuxième couche de diffusion poreuse (35) étant disposée adjacente à deuxième microcellule (38) et ayant une deuxième porosité différente de la première porosité.

2. Système d'apport de molécules actives selon la revendication 1, la couche de scellement hydrophobe comprenant un polyisobutylène, un polyéthylène, un polyuréthane, une polycaprolactone ou un polysiloxane.

3. Système d'apport de molécules actives selon la revendication 1, la couche de diffusion poreuse comprenant un acrylate, un méthacrylate, un polycarbonate, un poly(alcool vinylique), une cellulose, un poly(N-isopropylacrylamide) (PNIPAAm), un poly(acide lactique-co-glycolique) (PLGA), un poly(chlorure de vinylidène), l'acrylonitrile, un nylon amorphe, un polyester orienté, un téréphtalate, un poly(chlorure de vinyle), un polyéthylène, un polypropylène, un polybutylène, un polyisobutylène ou un polystyrène.

4. Système d'apport de molécules actives selon la revendication 1, chaque couche de diffusion poreuse (34, 35, 36) ayant une taille moyenne de pores comprise entre 10 nm et 100 µm.

5. Système d'apport de molécules actives selon la revendication 1, la molécule active hydrophile étant un composé pharmaceutique, un composé aromatique ou comprenant des acides nucléiques ou des acides aminés.

6. Système d'apport de molécules actives selon la revendication 1, chacune de la pluralité de microcellules ayant un volume supérieur à 100 nL.

7. Système d'apport de molécules actives selon la revendication 1, la formulation aqueuse comprenant plus d'un type de molécule active hydrophile.

8. Système d'apport de molécules actives selon la revendication 1, les première et deuxième formulations aqueuses étant différentes en ce qui concerne la concentration de la molécule active.

9. Système d'apport de molécules actives selon la revendication 1, comprenant en outre une couverture encapsulante qui encapsule le système d'apport de molécules actives.

10. Système d'apport de molécules actives selon la revendication 1, comprenant en outre une couche de support en contact avec la couche d'adhésif.

11. Système d'apport de molécules actives selon la revendication 1, la formulation aqueuse comprenant de plus un agent épaississant.

12. Système d'apport de molécules actives selon la revendication 11, l'agent épaississant étant un polymère.

13. Système d'apport de molécules actives selon la revendication 12, le polymère étant un copolymère d'éthylène et d'alcool vinylique.
